# EUROPÄISCHE PATENTSCHRIFT

(11) **EP 1 238 060 B1**
(45) Veröffentlichungstag und Bekanntmachung des Hinweises auf die Patenterteilung: **07.04.2004**
(21) Anmeldenummer: 00990521.7
(22) Anmeldetag: 08.12.2000
(51) Int. Cl.: C12N 5/06, C12N 5/00, A61L 27/60

(54) **VERFAHREN ZUR HERSTELLUNG EINES HAUTIMPLANTATS UND NACH DEM VERFAHREN HERGESTELLTES IMPLANTAT**
METHOD FOR PRODUCTION OF A SKIN GRAFT AND THE GRAFT PRODUCED THEREBY
PROCEDE ET REALISATION D'UN IMPLANT DE PEAU ET IMPLANT REALISE SELON LEDIT PROCEDE

(30) Priorität: 11.12.1999 DE 19959750
(43) Veröffentlichungstag der Anmeldung: 11.09.2002
(73) Patentinhaber: Si4Health GmbH, 93053 Regensburg (DE)
(72) Erfinder: Thierauf, Axel, 84066 Mallersdorf-Pfaffenberg (DE)
(74) Vertreter: Dehmel, Albrecht, Dr.
(86) Internationale Anmeldenummer: PCT/DE2000/004406
(87) Internationale Veröffentlichungsnummer: WO 2001/042428

(56) Entgegenhaltungen:
- DE-C- 19 609 551
- US-A- 5 650 164
- US-A- 5 968 546

## Beschreibung

Die Erfindung richtet sich auf ein Verfahren zur Herstellung eines Eigenhaut-Implantats, wobei man Hautzellen auf die Oberfläche einer Nährlösung aufbringt und wachsen läßt.

Hautimplantate werden insbesondere bei Opfern von Verbrennungen benötigt, deren Haut teilweise durch die Verbrennung zerstört ist bzw. bei sogenannten offenen Wunden, wie sie bei Beingewüren oft vorkommen. Es wird geschätzt, daß hiervon weltweit ca. 12 Mio. Menschen betroffen sind. Zur Herstellung von Hautimplantaten ist es bekannt, Zellen der Oberhaut des Patienten, sogenannte Keratinozyten, zu isolieren und in einer Nährlösung zum Wachsen anzuregen. Nach einigen Wochen entsteht hieraus ein sogenannter geschlossener Zellrasen (sheet graft), der auf Reste der Lederhaut verpflanzt werden kann. Möglich ist es auch, die so gewonnene Haut auf Leichenhaut zu verpflanzen, die von körpereigenen Zellen dann teilweise durchdrungen wird und eine Art Ersatz-Lederhaut bildet. Es wurde auch eine Unterlage aus Vorhäuten von Embryos entwickelt, die eine Fläche von 8 x 10 cm ergibt.

Noch schwieriger als die Züchtung von Keratinozyten ist diejenige von Dermis-Zellen, also Zellen der Lederhaut, oder von Fibroblasten. Wegen des langsamen Wachstum dieser Zellen dauert es mehrere Wochen, bis auch nur ein Quadratzentimeter Haut gezüchtet ist.

Die vorstehend beschriebenen Hautimplantate weisen zwei ausgeprägte Nachteile auf, nämlich zum einen die ganz erheblichen Kosten für deren Herstellung aufgrund des langsamen Wachstums und zum anderen deren Empfindlichkeit gegenüber mechanischer Beanspruchung bei der Transplantation.

Hiervon ausgehend liegt der Erfindung die Aufgabe zugrunde, ein Verfahren zur Herstellung eines Haut-Implantats anzugeben, welches die Nachteile herkömmlicher Verfahren vermeidet.

Diese Aufgabe wird erfindungsgemäß dadurch gelöst, daß auf die Nährlösung ein Flächenelement, insbesondere ein Vlies oder Gewebe aus biokompatiblem, biologisch degradierbarem und/oder biologisch resorbierbarem Material aufgelegt wird, längs dessen die auf die Nährlösung aufgebrachten Hautzellen wachsen und sich vermehren.

Das erfindungsgemäße Vorgehen hat zur Folge, daß der "Hautrasen" zu einem ausschließlich flächigen, gerichteten Wachstum angeregt wird und ein clusterartiges Wachstum in die Tiefe vermieden wird. Hierdurch wird erreicht, daß das Wachstum ausschließlich der Flächenvergrößerung zugute kommt und damit wird eine deutlich gesteigerte Wachstumsrate erzielt, was nicht nur die Zeiten verkürzt, bis einem Patienten gerechnet vom Zeitpunkt der Entnahme der Hautzellen ein Hautimplantat transplantiert werden kann und sich somit kostensenkend auswirkt, sondern darüber hinaus wird durch das flächige Stützelement auch die mechanische Festigkeit erheblich verbessert, was sowohl bei der Transplantation als auch im implantierten Zustand deutliche Vorteile bringt.

Vorzugsweise ist vorgesehen, daß das Flächenelement, insbesondere in Form eines Vlieses oder Gewebes, aus biologisch degradierbaren und/oder biologisch resorbierbaren Fasern besteht, die man dadurch erhält, daß man aus einer Spinnmasse Fäden zieht und diese gegebenenfalls trocknet, wobei die Spinnmasse eine oder mehrere partiell oder vollständig hydrolytisch kondensierte Verbindungen des Siliciums enthält, die sich durch hydrolytische Kondensation ableiten von Monomeren der allgemeinen Formel SiX₄, in der die Reste X gleich oder verschieden sind und Hydroxy, Wasserstoff, Halogen, Amino, Alkoxy, Alcyloxy, Alkylcarbonyl oder Alkoxycarbonyl bedeuten und sich von Alkyl-Resten ableiten, die gegebenenfalls substituierte geradkettige, verzweigte oder cyclische Reste mit 1 bis 20 Kohlenstoff-Atomen, bevorzugt mit 1 bis 10 Kohlenstoff-Atomen, darstellen und durch Sauerstoff- oder Schwefel-Atome oder durch Amino-Gruppen unterbrochen sein können.

Eine derartige Faser wird hinsichtlich ihrer Eigenschaften und ihrer Herstellung in DE 196 09 551 C1 ausführlich beschrieben.

Solche Fasern sind biologisch degradierbar und biologisch resorbierbar und lösen sich in schwach basischen, körperähnlichen Flüssigkeiten mit Abbauraten zwischen 10 und 100 nm Faserradius pro Tag auf, wobei die Abbaurate mit der Zahl der Silanol-Gruppen der Faser korreliert ist.

Diese Fasern weisen dementsprechend gegenüber biologisch degradierbaren Osteosynthesematerialien, die z.B. aus Derivaten der Milchsäure (Polylactide) bestehen, den Vorteil auf, daß sich im menschlichen Organismus im Bereich der Fasern kein saures Milieu entsteht, was zu Unverträglichkeiten oder sogar zur Krebsbildung führen kann.

Weiterhin bieten diese Fasertypen die Möglichkeiten der Infiltration mit Medikamenten, die an den endständigen OH-Gruppen chemisch gebunden werden können.

Bei der Herstellung dieser Fasern haben diese nach vollständiger Hydrolyse der Spinnmasse die formale chemische Zusammensetzung Siₙ(OH)₂ₓO₂ₙ₋ₓ. Durch den Grad der Kondensation kann die Abbaurate und die Resorbierbarkeit derartiger Fasern gezielt gesteuert und den Erfordernissen des jeweiligen Anwendungsfalles angepaßt werden. Je geringer das Ausmaß der Kondensation, d.h. je höher die Anzahl der verbliebenen OH-Gruppen, desto höher die Abbaurate und desto größer ist die Resorbierbarkeit der erfindungsgemäßen Fasern.

In der DE 196 09 551 C1 wurde bereits in Betracht gezogen, diese Fasern als Verstärkung für Implantate heranzuziehen. Demgegenüber werden erfindungsgemäß Flächenelemente aus solchen Fasern eingesetzt, um gezielt ein flächiges und damit beschleunigtes Wachstum von Hautzellen zu induzieren, gegebenenfalls unter Einsatz von infiltrierten Medikamenten, und der an sich bekannte Verstärkungseffekt bei der Transplantation und beim fertigen Implantat ergibt sich als zusätzlicher Vorteil.

Mit Hilfe des erfindungsgemäßen Verfahrens ist es möglich, gewerbliche Implantat-Züchtung zu betreiben, wobei Kliniken oder auch Ärzte Patienten, die einer Hautransplantation bedürfen, z.B. infolge von Brandverletzungen oder auch infolge des Wundliegens, eine Hautprobe zu entnehmen, die dem jeweiligen Institut zur Züchtung eines Eigenhaut-Implantats übermittelt wird. Das Institut züchtet dann mit einer gegenüber herkömmlichen Wachstumszeiten deutlich verkürzten Zeitspanne ein Implantat gewünschter Größe und liefert dieses an den Arzt oder die Klinik, wo die Implantation vorgenommen wird.

## Patentansprüche

1. Verfahren zur Herstellung eines Hautimplantats, wobei man Hautzellen auf die Oberfläche einer Nährlösung aufbringt und wachsen läßt, **dadurch gekennzeichnet, daß** auf die Nährlösung ein Flächenelement aus biokompatiblen, biologisch degradierbaren und/oder biologisch resorbierbaren Fasern, die man dadurch erhält, daß man aus einer Spinnmasse Fäden zieht und diese trocknet, wobei die Spinnmasse eine oder mehrere partiell oder vollständig hydrolytisch kondensierte Verbindungen des Siliciums enthält, die sich durch hydrolytische Kondensation ableiten von Monomeren der allgemeinen Formel SiX₄, in der die Reste X gleich oder verschieden sind und Hydroxy, Wasserstoff, Halogen, Amino, Alkoxy, Alkyloxy, Alkylcarbonyl oder Alkoxycarbonyl bedeuten und sich von Alkyl-Resten ableiten und durch Sauerstoff- oder Schwefel-Atome oder durch Amino-Gruppen unterbrochen sein können, aufgelegt wird, längs dessen die auf die Nährlösung aufgebrachten Hautzellen wachsen und sich vermehren, wobei die Fasern des Flächenelements einen Durchmesser von 5 bis 20 µm aufweisen.

2. Hautimplantat bestebend aus Hautzellen und ein Flächen element, **dadurch gekennzeichnet, daß** das Flächenelement aus biokompatiblen, biologisch degradierbaren und/oder biologisch resorbierbaren Fasern besteht, die man dadurch erhält, daß man aus einer Spinnmasse Fäden zieht und diese trocknet, wobei die Spinnmasse eine oder mehrere partiell oder vollständig hydrolytisch kondensierte Verbindungen des Siliciums enthält, die sich durch hydrolytische Kondensation ableiten von Monomeren der allgemeinen Formel SiX₄, in der die Reste X gleich oder verschieden sind und Hydroxy, Wasserstoff, Halogen, Amino, Alkoxy, Alkyloxy, Alkylcarbonyl oder Alkoxycarbonyl bedeuten und sich von Alkyl-Resten ableiten und durch Sauerstoff- oder Schwefel-Atome oder durch Amino-Gruppen unterbrochen sein können, längs dessen die Hautzellen wachsen und sich vermehren, wobei die Fasern des Flächenelements einen Durchmesser von 5 bis 20 µm aufweisen.

## Claims

1. A method for producing a skin implant, wherein skin cells are applied to the surface of a nutrient solution and are left to grow, **characterised in that** applied to the nutrient solution is an area element made of bio-compatible, bio-degradable and/or bio-reabsorbable fibres which are obtained **in that** fibres are drawn from a spinning mass which fibres are then dried, wherein the spinning mass contains one or several partially or completely hydrolytically-condensed silicon compounds which by hydrolytic condensation are derived from monomers of the general formula SiX₄, in which the residues X are identical or different, with X meaning hydroxy, hydrogen, halogen, amino, alkoxy, alkyloxy, alkylcarbonyl or alcoxycarbonyl, and are derived from alkyl residues and can be interrupted by oxygen atoms or sulphur atoms or by amino groups - along which area element the skin cells which have been applied to the nutrient solution grow and multiply, wherein the diameter of the fibres of the area element ranges from 5 to 20 µm.

2. A skin implant consisting of skin cells and an area element, **characterised in that** the area element is made of bio-compatible, bio-degradable and/or bio-reabsorbable fibres which are obtained **in that** fibres are drawn from a spinning mass which fibres are then dried, wherein the spinning mass contains one or several partially or completely hydrolytically-condensed silicon compounds which by hydrolytic condensation are derived from monomers of the general formula SiX₄, in which the residues X are identical or different, with X meaning hydroxy, hydrogen, halogen, amino, alkoxy, alkyloxy, alkylcarbonyl or alcoxycarbonyl, and are derived from alkyl residues and can be interrupted by oxygen atoms or sulphur atoms or by amino groups - along which area element the skin cells grow and multiply, wherein the diameter of the fibres of the area element ranges from 5 to 20 µm.

## Revendications

1. Procédé de préparation d'un implant cutané, dans lequel on dépose des cellules cutanées à la surface d'une solution nutritive et on les laisse croître, **caractérisé en ce qu'**on applique sur la solution nutritive un élément de surface en fibres biocompatibles, biodégradables et/ou bio-résorbables, qui sont obtenues **en ce que** l'on tire des fils à partir d'une masse de filage et **en ce qu'**on les sèche, la masse de filage contenant un ou plusieurs composés du silicium partiellement ou totalement condensés par hydrolyse, qui sont dérivés par condensation hydrolytique de monomères de formule générale SiX₄ ; dans laquelle les restes X sont identiques ou différents et représentent hydroxy, hydrogène, halogène, amino, alcoxy, alkyloxy, alkylcarbonyle ou alcoxycarbonyle et sont dérivés de restes alkyle et peuvent être interrompus par des atomes d'oxygène ou de soufre ou par des groupes amino, le long desquelles fibres les cellules appliquées sur la solution nutritive croissent et se multiplient, les fibres des éléments de surface présentant un diamètre de 5 à 20 µm.

2. Implant cutané comprenant des cellules cutanées et un élément de surface, **caractérisé en ce que** l'élément de surface comprend des fibres biocompatibles, biodégradables et/ou bio-résorbables qui sont obtenues **en ce que** l'on tire des fils à partir d'une masse de filage et **en ce qu'**on les sèche, la masse de filage contenant un ou plusieurs composés du silicium partiellement ou totalement condensés par hydrolyse, qui sont dérivés par condensation hydrolytique de monomères de formule générale SiX₄ ; dans laquelle les restes X sont identiques ou différents et représentent hydroxy, hydrogène, halogène, amino, alcoxy, alkyloxy, alkylcarbonyle ou alcoxycarbonyle et sont dérivés de restes alkyle et peuvent être interrompus par des atomes d'oxygène ou de soufre ou par des groupes amino, le long desquelles fibres les cellules appliquées sur la solution nutritive croissent et se multiplient, les fibres des éléments de surface présentant un diamètre de 5 à 20 µm.
